# EUROPEAN PATENT APPLICATION

(11) **EP 1 932 910 A1**
(43) Date of publication of application: **18.06.2008**
(21) Application number: 06783178.4
(22) Date of filing: 05.09.2006
(51) Int. Cl.: C12N 15/09, C12N 1/21, C12P 7/56

(54) **PROCESS FOR PRODUCTION OF LACTIC ACID**

(30) Priority: 05.09.2005 JP 2005255902
(71) Applicant: KYOWA HAKKO KOGYO CO., LTD., Chiyoda-ku, Tokyo 100-8185 (JP)
(72) Inventor: ITO, Mikito, Machida-shi, Tokyo 194-8533 (JP); MASUDA, Kimie, Machida-shi, Tokyo 194-0032 (JP); MORI, Hideo, Machida-shi, Tokyo 194-0021 (JP); KATO, Makiko, Machida-shi, Tokyo 194-0022 (JP)
(74) Representative: Bohmann, Armin K.
(86) International application number: PCT/JP2006/317489
(87) International publication number: WO 2007/029664

(57) **Abstract**

The present invention provides microorganisms, in which the activity of 4-hydroxybenzoate polyprenyltransferase or 2-octaprenylphenol→2-octaprenyl-6-methoxyphenol flavin reductase is reduced or lost, and which have an ability to produce lactic acid, in particular, microorganisms comprising a chromosomal DNA in which a gene encoding a protein having 4-hydroxybenzoate polyprenyltransferase activity or a protein having 2-octaprenylphenol→2-octaprenyl-6-methoxyphenol flavin reductase activity is partially or completely defective; and a process for producing lactic acid using the microorganisms.

<110> KYOWA HAKKO KOGYO CO., LTD.

<120> Process for production of lactic acid

<130> K 10007 EP

<150> JP2005-255902
<151> 2005-09-05

<160> 21

<170> PatentIn Ver. 2.1

<210> 1
<211> 290
<212> PRT
<213> Escherichia coli

<400> 1
Met Glu Trp Ser Leu Thr Gln Asn Lys Leu Leu Ala Phe His Arg Leu
  1               5                  10                  15

Met Arg Thr Asp Lys Pro Ile Gly Ala Leu Leu Leu Leu Trp Pro Thr
             20                  25                  30

Leu Trp Ala Leu Trp Val Ala Thr Pro Gly Val Pro Gln Leu Trp Ile
         35                  40                  45

Leu Ala Val Phe Val Ala Gly Val Trp Leu Met Arg Ala Ala Gly Cys
     50                  55                  60

Val Val Asn Asp Tyr Ala Asp Arg Lys Phe Asp Gly His Val Lys Arg
 65                  70                  75                  80

Thr Ala Asn Arg Pro Leu Pro Ser Gly Ala Val Thr Glu Lys Glu Ala
                 85                  90                  95

Arg Ala Leu Phe Val Val Leu Val Leu Ile Ser Phe Leu Leu Val Leu
            100                 105                 110

Thr Leu Asn Thr Met Thr Ile Leu Leu Ser Ile Ala Ala Leu Ala Leu
        115                 120                 125

Ala Trp Val Tyr Pro Phe Met Lys Arg Tyr Thr His Leu Pro Gln Val
    130                 135                 140

Val Leu Gly Ala Ala Phe Gly Trp Ser Ile Pro Met Ala Phe Ala Ala
145                 150                 155                 160

Val Ser Glu Ser Val Pro Leu Ser Cys Trp Leu Met Phe Leu Ala Asn
                165                 170                 175

Ile Leu Trp Ala Val Ala Tyr Asp Thr Gln Tyr Ala Met Val Asp Arg
            180                 185                 190

Asp Asp Asp Val Lys Ile Gly Ile Lys Ser Thr Ala Ile Leu Phe Gly
        195                 200                 205

Gln Tyr Asp Lys Leu Ile Ile Gly Ile Leu Gln Ile Gly Val Leu Ala
    210                 215                 220

Leu Met Ala Ile Ile Gly Glu Leu Asn Gly Leu Gly Trp Gly Tyr Tyr
225                 230                 235                 240

Trp Ser Ile Leu Val Ala Gly Ala Leu Phe Val Tyr Gln Gln Lys Leu
                245                 250                 255

Ile Ala Asn Arg Glu Arg Glu Ala Cys Phe Lys Ala Phe Met Asn Asn
            260                 265                 270

Asn Tyr Val Gly Leu Val Leu Phe Leu Gly Leu Ala Met Ser Tyr Trp
        275                 280                 285

His Phe


<210> 2
<211> 546
<212> PRT
<213> Escherichia coli

<400> 2
Met Thr Pro Gly Glu Val Arg Arg Leu Tyr Phe Ile Ile Arg Thr Phe
  1               5                  10                  15

Leu Ser Tyr Gly Leu Asp Glu Leu Ile Pro Lys Met Arg Ile Thr Leu
             20                  25                  30

Pro Leu Arg Leu Trp Arg Tyr Ser Leu Phe Trp Met Pro Asn Arg His
         35                  40                  45

Lys Asp Lys Leu Leu Gly Glu Arg Leu Arg Leu Ala Leu Gln Glu Leu
     50                  55                  60

Gly Pro Val Trp Ile Lys Phe Gly Gln Met Leu Ser Thr Arg Arg Asp
 65                  70                  75                  80

Leu Phe Pro Pro His Ile Ala Asp Gln Leu Ala Leu Leu Gln Asp Lys
                 85                  90                  95

Val Ala Pro Phe Asp Gly Lys Leu Ala Lys Gln Gln Ile Glu Ala Ala
            100                 105                 110

Met Gly Gly Leu Pro Val Glu Ala Trp Phe Asp Asp Phe Glu Ile Lys
        115                 120                 125

Pro Leu Ala Ser Ala Ser Ile Ala Gln Val His Thr Ala Arg Leu Lys
    130                 135                 140

Ser Asn Gly Lys Glu Val Val Ile Lys Val Ile Arg Pro Asp Ile Leu
145                 150                 155                 160

Pro Val Ile Lys Ala Asp Leu Lys Leu Ile Tyr Arg Leu Ala Arg Trp
                165                 170                 175

Val Pro Arg Leu Leu Pro Asp Gly Arg Arg Leu Arg Pro Thr Glu Val
            180                 185                 190

Val Arg Glu Tyr Glu Lys Thr Leu Ile Asp Glu Leu Asn Leu Leu Arg
        195                 200                 205

Glu Ser Ala Asn Ala Ile Gln Leu Arg Arg Asn Phe Glu Asp Ser Pro
    210                 215                 220

Met Leu Tyr Ile Pro Glu Val Tyr Pro Asp Tyr Cys Ser Glu Gly Met
225                 230                 235                 240

Met Val Met Glu Arg Ile Tyr Gly Ile Pro Val Ser Asp Val Ala Ala
                245                 250                 255

Leu Glu Lys Asn Gly Thr Asn Met Lys Leu Leu Ala Glu Arg Gly Val
            260                 265                 270

Gln Val Phe Phe Thr Gln Val Phe Arg Asp Ser Phe Phe His Ala Asp
        275                 280                 285

Met His Pro Gly Asn Ile Phe Val Ser Tyr Glu His Pro Glu Asn Pro
    290                 295                 300

Lys Tyr Ile Gly Ile Asp Cys Gly Ile Val Gly Ser Leu Asn Lys Glu
305                 310                 315                 320

Asp Lys Arg Tyr Leu Ala Glu Asn Phe Ile Ala Phe Phe Asn Arg Asp
                325                 330                 335

Tyr Arg Lys Val Ala Glu Leu His Val Asp Ser Gly Trp Val Pro Pro
            340                 345                 350

Asp Thr Asn Val Glu Glu Phe Glu Phe Ala Ile Arg Thr Val Cys Glu
        355                 360                 365

Pro Ile Phe Glu Lys Pro Leu Ala Glu Ile Ser Phe Gly His Val Leu
    370                 375                 380

Leu Asn Leu Phe Asn Thr Ala Arg Arg Phe Asn Met Glu Val Gln Pro
385                 390                 395                 400

Gln Leu Val Leu Leu Gln Lys Thr Leu Leu Tyr Val Glu Gly Val Gly
                405                 410                 415

Arg Gln Leu Tyr Pro Gln Leu Asp Leu Trp Lys Thr Ala Lys Pro Phe
            420                 425                 430

Leu Glu Ser Trp Ile Lys Asp Gln Val Gly Ile Pro Ala Leu Val Arg
        435                 440                 445

Ala Phe Lys Glu Lys Ala Pro Phe Trp Val Glu Lys Met Pro Glu Leu
    450                 455                 460

Pro Glu Leu Val Tyr Asp Ser Leu Arg Gln Gly Lys Tyr Leu Gln His
465                 470                 475                 480

Ser Val Asp Lys Ile Ala Arg Glu Leu Gln Ser Asn His Val Arg Gln
                485                 490                 495

Gly Gln Ser Arg Tyr Phe Leu Gly Ile Gly Ala Thr Leu Val Leu Ser
            500                 505                 510

Gly Thr Phe Leu Leu Val Ser Arg Pro Glu Trp Gly Leu Met Pro Gly
        515                 520                 525

Trp Leu Met Ala Gly Gly Leu Ile Ala Trp Phe Val Gly Trp Arg Lys
    530                 535                 540

Thr Arg
545

<210> 3
<211> 1170
<212> DNA
<213> Escherichia coli

<400> 3
gagtctcgtt actggttacg tgaaattttg ttatgtgccg atggtgaacc gtggcttgcc 60

ggtcgtaccg tcgttcctgt gtcaacgtta agcgggccgg agctggcgtt acaaaaattg 120

ggtaaaacgc cgttaggacg ctatctgttc acatcatcga cattaacccg ggactttatt 180

gagataggcc gtgatgccgg gctgtggggg cgacgttccc gcctgcgatt aagcggtaaa 240

ccgctgttgc taacagaact gtttttaccg gcgtcaccgt tgtactaaga ggaaaaaaat 300

atg gag tgg agt ctg acg cag aat aag ctg ctg gcg ttt cat cgc tta 348
Met Glu Trp Ser Leu Thr Gln Asn Lys Leu Leu Ala Phe His Arg Leu
1               5                   10                  15

atg cgt acg gat aag cca att ggc gcg tta ctg ctg ctc tgg cca aca 396
Met Arg Thr Asp Lys Pro Ile Gly Ala Leu Leu Leu Leu Trp Pro Thr
            20                  25                  30

tta tgg gcg ttg tgg gtg gcg aca ccg ggc gtt ccc cag ctc tgg atc 444
Leu Trp Ala Leu Trp Val Ala Thr Pro Gly Val Pro Gln Leu Trp Ile
        35                  40                  45

ctg gcg gtg ttt gtc gcg ggt gtc tgg ctg atg cgc gct gcc gga tgt 492
Leu Ala Val Phe Val Ala Gly Val Trp Leu Met Arg Ala Ala Gly Cys
    50                  55                  60

gtg gtg aat gat tat gct gac cgc aag ttt gat ggt cat gtt aag cgc 540
Val Val Asn Asp Tyr Ala Asp Arg Lys Phe Asp Gly His Val Lys Arg
65                  70                  75                  80

acg gcg aac cga cca ctt ccc agc ggc gcg gta aca gag aaa gag gcg 588
Thr Ala Asn Arg Pro Leu Pro Ser Gly Ala Val Thr Glu Lys Glu Ala
                85                  90                  95

cgc gcg ctg ttt gtc gtg ctg gta ctg att tcg ttt tta ctg gtg ctg     636
Arg Ala Leu Phe Val Val Leu Val Leu Ile Ser Phe Leu Leu Val Leu
            100                 105                 110

acg ctg aat acg atg acc att ctg ttg tcg att gcc gcg cta gcg ctg     684
Thr Leu Asn Thr Met Thr Ile Leu Leu Ser Ile Ala Ala Leu Ala Leu
        115                 120                 125

gcg tgg gtg tac ccg ttt atg aag cgg tat acc cat cta ccg caa gtg     732
Ala Trp Val Tyr Pro Phe Met Lys Arg Tyr Thr His Leu Pro Gln Val
    130                 135                 140

gtg ctg ggc gcg gcg ttt ggc tgg tcg att cca atg gct ttt gcc gct     780
Val Leu Gly Ala Ala Phe Gly Trp Ser Ile Pro Met Ala Phe Ala Ala
145                 150                 155                 160

gtg agt gag tcg gtg cca ttg agt tgc tgg tta atg ttc ctc gcc aat     828
Val Ser Glu Ser Val Pro Leu Ser Cys Trp Leu Met Phe Leu Ala Asn
                165                 170                 175

att ctc tgg gcg gtg gct tac gac acg cag tat gcg atg gtt gac cgc     876
Ile Leu Trp Ala Val Ala Tyr Asp Thr Gln Tyr Ala Met Val Asp Arg
            180                 185                 190

gat gat gat gtg aag att ggc att aaa tcc acg gca atc ctg ttc ggc     924
Asp Asp Asp Val Lys Ile Gly Ile Lys Ser Thr Ala Ile Leu Phe Gly
        195                 200                 205

caa tac gat aaa ttg att att ggt att ttg cag att ggc gta ctg gca     972
Gln Tyr Asp Lys Leu Ile Ile Gly Ile Leu Gln Ile Gly Val Leu Ala
    210                 215                 220

ctg atg gcg atc atc ggt gag tta aat ggc tta ggc tgg gga tat tac     1020
Leu Met Ala Ile Ile Gly Glu Leu Asn Gly Leu Gly Trp Gly Tyr Tyr
225                 230                 235                 240

tgg tca att ctg gtg gct ggc gcg ctg ttt gtt tat caa caa aaa ctg     1068
Trp Ser Ile Leu Val Ala Gly Ala Leu Phe Val Tyr Gln Gln Lys Leu
                245                 250                 255

att gcc aac cgc gag cgt gaa gcc tgc ttt aaa gca ttt atg aat aat     1116
Ile Ala Asn Arg Glu Arg Glu Ala Cys Phe Lys Ala Phe Met Asn Asn
            260                 265                 270

aac tat gtt ggt ctg gta cta ttt tta ggg ctg gca atg agt tac tgg     1164
Asn Tyr Val Gly Leu Val Leu Phe Leu Gly Leu Ala Met Ser Tyr Trp
        275                 280                 285

cat ttc                                                             1170
His Phe
    290

<210> 4
<211> 1938
<212> DNA
<213> Escherichia coli

<400> 4
tattcaggtg gtgcaaaact tcgttgcgct ggcagatctg gcagagttcg accctgcgga 60

actgctggcc ccttataccg gtgatatcgc cgctgaagga atcagcaaag ccatgcgcgg 120

aggcgcaaag ttcctgcatc acggcattaa gcgccagcaa cgttatgtgg cggaagccat 180

tactgaagag tggcgtatgg cacccggtcc gcttgaagtg gcctggtttg cggaagagac 240

ggctgccgtc gagcgtgctg ttgatgccct gaccaaacgg ctggaaaaac tggaggctaa 300

atg acg cca ggt gaa gta cgg cgc cta tat ttc atc att cgc act ttt 348
Met Thr Pro Gly Glu Val Arg Arg Leu Tyr Phe Ile Ile Arg Thr Phe
1               5                   10                  15

tta agc tac gga ctt gat gaa ctg atc ccc aaa atg cgt atc acc ctg 396
Leu Ser Tyr Gly Leu Asp Glu Leu Ile Pro Lys Met Arg Ile Thr Leu
            20                  25                  30

ccg cta cgg cta tgg cga tac tca tta ttc tgg atg cca aat cgg cat 444
Pro Leu Arg Leu Trp Arg Tyr Ser Leu Phe Trp Met Pro Asn Arg His
        35                  40                  45

aaa gac aaa ctt tta ggt gag cga cta cga ctg gcc ctg caa gaa ctg 492
Lys Asp Lys Leu Leu Gly Glu Arg Leu Arg Leu Ala Leu Gln Glu Leu
    50                  55                  60

ggg ccg gtt tgg atc aag ttc ggg caa atg tta tca acc cgc cgc gat 540
Gly Pro Val Trp Ile Lys Phe Gly Gln Met Leu Ser Thr Arg Arg Asp
65                  70                  75                  80

ctt ttt cca ccg cat att gcc gat cag ctg gcg tta ttg cag gac aaa 588
Leu Phe Pro Pro His Ile Ala Asp Gln Leu Ala Leu Leu Gln Asp Lys
                85                  90                  95

gtt gct ccg ttt gat ggc aag ctg gcg aag cag cag att gaa gct gca 636
Val Ala Pro Phe Asp Gly Lys Leu Ala Lys Gln Gln Ile Glu Ala Ala
            100                 105                 110

atg ggc ggc ttg ccg gta gaa gcg tgg ttt gac gat ttt gaa atc aag 684
Met Gly Gly Leu Pro Val Glu Ala Trp Phe Asp Asp Phe Glu Ile Lys
        115                 120                 125

ccg ctg gct tct gct tct atc gcc cag gtt cat acc gcg cga ttg aaa 732
Pro Leu Ala Ser Ala Ser Ile Ala Gln Val His Thr Ala Arg Leu Lys
    130                 135                 140

tcg aat ggt aaa gag gtg gtg att aaa gtc atc cgc ccg gat att ttg 780
Ser Asn Gly Lys Glu Val Val Ile Lys Val Ile Arg Pro Asp Ile Leu
145                 150                 155                 160

ccg gtt att aaa gcg gat ctg aaa ctt atc tac cgt ctg gct cgc tgg 828
Pro Val Ile Lys Ala Asp Leu Lys Leu Ile Tyr Arg Leu Ala Arg Trp
                165                 170                 175

gtg ccg cgt ttg ctg ccg gat ggt cgc cgt ctg cgc cca acc gaa gtg 876

Val Pro Arg Leu Leu Pro Asp Gly Arg Arg Leu Arg Pro Thr Glu Val
            180                 185                 190

gtg cgc gag tac gaa aag aca ttg att gat gaa ctg aat ttg ctg cgg     924
Val Arg Glu Tyr Glu Lys Thr Leu Ile Asp Glu Leu Asn Leu Leu Arg
        195                 200                 205

gaa tct gcc aac gcc att cag ctt cgg cgc aat ttt gaa gac agc ccg     972
Glu Ser Ala Asn Ala Ile Gln Leu Arg Arg Asn Phe Glu Asp Ser Pro
    210                 215                 220

atg ctc tac atc ccg gaa gtt tac cct gac tat tgt agt gaa ggg atg     1020
Met Leu Tyr Ile Pro Glu Val Tyr Pro Asp Tyr Cys Ser Glu Gly Met
225                 230                 235                 240

atg gtg atg gag cgc att tac ggc att ccg gtg tct gat gtt gcg gcg     1068
Met Val Met Glu Arg Ile Tyr Gly Ile Pro Val Ser Asp Val Ala Ala
                245                 250                 255

ctg gag aaa aac ggc act aac atg aaa ttg ctg gcg gaa cgc ggc gtg     1116
Leu Glu Lys Asn Gly Thr Asn Met Lys Leu Leu Ala Glu Arg Gly Val
            260                 265                 270

cag gtg ttc ttc act cag gtc ttt cgc gac agc ttt ttc cat gcc gat     1164
Gln Val Phe Phe Thr Gln Val Phe Arg Asp Ser Phe Phe His Ala Asp
        275                 280                 285

atg cac cct ggc aac atc ttc gta agc tat gaa cac ccg gaa aac ccg     1212
Met His Pro Gly Asn Ile Phe Val Ser Tyr Glu His Pro Glu Asn Pro
    290                 295                 300

aaa tat atc ggc att gat tgc ggg att gtt ggc tcg cta aac aaa gaa     1260
Lys Tyr Ile Gly Ile Asp Cys Gly Ile Val Gly Ser Leu Asn Lys Glu
305                 310                 315                 320

gat aaa cgc tat ctg gca gaa aac ttt atc gcc ttc ttt aat cgc gac     1308
Asp Lys Arg Tyr Leu Ala Glu Asn Phe Ile Ala Phe Phe Asn Arg Asp
                325                 330                 335

tat cgc aaa gtg gca gag cta cac gtc gat tct ggc tgg gtg cca cca     1356
Tyr Arg Lys Val Ala Glu Leu His Val Asp Ser Gly Trp Val Pro Pro
            340                 345                 350

gat acc aac gtt gaa gag ttc gaa ttt gcc att cgt acg gtc tgt gaa     1404
Asp Thr Asn Val Glu Glu Phe Glu Phe Ala Ile Arg Thr Val Cys Glu
        355                 360                 365

cct atc ttt gag aaa ccg ctg gcc gaa att tcg ttt gga cat gta ctg     1452
Pro Ile Phe Glu Lys Pro Leu Ala Glu Ile Ser Phe Gly His Val Leu
    370                 375                 380

tta aat ctg ttt aat acg gcg cgt cgc ttc aat atg gaa gtg cag ccg     1500
Leu Asn Leu Phe Asn Thr Ala Arg Arg Phe Asn Met Glu Val Gln Pro
385                 390                 395                 400

caa ctg gtg tta ctc cag aaa acc ctg ctc tac gtc gaa ggg gta gga     1548
Gln Leu Val Leu Leu Gln Lys Thr Leu Leu Tyr Val Glu Gly Val Gly

405                 410                 415

cgc cag ctt tat ccg caa ctc gat tta tgg aaa acg gcg aag cct ttc     1596
Arg Gln Leu Tyr Pro Gln Leu Asp Leu Trp Lys Thr Ala Lys Pro Phe
            420                 425                 430

ctg gag tcg tgg att aaa gat cag gtc ggt att cct gcg ctg gtg aga     1644
Leu Glu Ser Trp Ile Lys Asp Gln Val Gly Ile Pro Ala Leu Val Arg
        435                 440                 445

gca ttt aaa gaa aaa gcg ccg ttc tgg gtc gaa aaa atg cca gaa ctg     1692
Ala Phe Lys Glu Lys Ala Pro Phe Trp Val Glu Lys Met Pro Glu Leu
    450                 455                 460

cct gaa ttg gtt tac gac agt ttg cgc cag ggc aag tat tta cag cac     1740
Pro Glu Leu Val Tyr Asp Ser Leu Arg Gln Gly Lys Tyr Leu Gln His
465                 470                 475                 480

agt gtt gat aag att gcc cgc gag ctt cag tca aat cat gta cgt cag     1788
Ser Val Asp Lys Ile Ala Arg Glu Leu Gln Ser Asn His Val Arg Gln
                485                 490                 495

gga caa tcg cgt tat ttt ctc gga att ggc gct acg tta gta tta agt     1836
Gly Gln Ser Arg Tyr Phe Leu Gly Ile Gly Ala Thr Leu Val Leu Ser
            500                 505                 510

ggc aca ttc ttg ttg gtc agc cga cct gaa tgg ggg ctg atg ccc ggc     1884
Gly Thr Phe Leu Leu Val Ser Arg Pro Glu Trp Gly Leu Met Pro Gly
        515                 520                 525

tgg tta atg gca ggt ggt ctg atc gcc tgg ttt gtc ggt tgg cgc aaa     1932
Trp Leu Met Ala Gly Gly Leu Ile Ala Trp Phe Val Gly Trp Arg Lys
    530                 535                 540

aca cgc                                                             1938
Thr Arg
545


<210> 5
<211> 50
<212> DNA
<213> Artificial Sequence

<220>
<223> Description of Artificial Sequence: Synthetic DNA

<400> 5
ttatttgccg actaccttgg tgatctgtgt ctcaaaatct ctgatgttac          50


<210> 6
<211> 50
<212> DNA
<213> Artificial Sequence

<220>

<223> Description of Artificial Sequence: Synthetic DNA

<400> 6
attcccctgc tcgcgcaggc tgggtttaga aaaactcatc gagcatcaaa          50


<210> 7
<211> 25
<212> DNA
<213> Artificial Sequence

<220>
<223> Description of Artificial Sequence: Synthetic DNA

<400> 7
ttatttgccg actaccttgg tgatc                                     25


<210> 8
<211> 25
<212> DNA
<213> Artificial Sequence

<220>
<223> Description of Artificial Sequence: Synthetic DNA

<400> 8
acccagcctg cgggagcagg ggaat                                     25


<210> 9
<211> 70
<212> DNA
<213> Artificial Sequence

<220>
<223> Description of Artificial Sequence: Synthetic DNA

<400> 9
ctgtttttac cggcgtcacc gttgtactaa gaggaaaaaa atatgttatt tgccgactac 60

cttggtgatc                                                      70


<210> 10
<211> 70
<212> DNA
<213> Artificial Sequence

<220>
<223> Description of Artificial Sequence: Synthetic DNA

<400> 10
acccagaaga aagccggatg atcatccggc ttttttacat catcaattcc cctgctcgcg 60

caggctgggt                                                      70

<210> 11
<211> 70
<212> DNA
<213> Artificial Sequence

<220>
<223> Description of Artificial Sequence: Synthetic DNA

<400> 11
tgttgatgcc ctgaccaaac ggctggaaaa actggaggct aaatgttatt tgccgactac 60

cttggtgatc 70


<210> 12
<211> 70
<212> DNA
<213> Artificial Sequence

<220>
<223> Description of Artificial Sequence: Synthetic DNA

<400> 12
ccgcattata cgttacacgg cccgccttga gcgatgaaaa aatcaattcc cctgctcgcg 60

caggctgggt 70


<210> 13
<211> 34
<212> DNA
<213> Artificial Sequence

<220>
<223> Description of Artificial Sequence: Synthetic DNA

<400> 13
gaagttccta tactttctag agaataggaa cttc 34


<210> 14
<211> 53
<212> DNA
<213> Artificial Sequence

<220>
<223> Description of Artificial Sequence: Synthetic DNA

<400> 14
ttatttgccg actaccttgg tgatcgatcc tttttaaccc atcacatata cct 53


<210> 15
<211> 45
<212> DNA
<213> Artificial Sequence

<220>
<223> Description of Artificial Sequence: Synthetic DNA

<400> 15
cgtgccgatc aacgtctcat atgccgcccg gtagtccggc ttctg 45

<210> 16
<211> 45
<212> DNA
<213> Artificial Sequence

<220>
<223> Description of Artificial Sequence: Synthetic DNA

<400> 16
cagaagccgg actaccgggc ggcatatgag acgttgatcg gcacg 45

<210> 17
<211> 45
<212> DNA
<213> Artificial Sequence

<220>
<223> Description of Artificial Sequence: Synthetic DNA

<400> 17
attcccctgc tcgcgcaggc tgggttgaat ttctgccatt catcc 45

<210> 18
<211> 68
<212> DNA
<213> Artificial Sequence

<220>
<223> Description of Artificial Sequence: Synthetic DNA

<400> 18
agtagcttaa atgtgattca acatcactgg agaaagtctt atgttatttg ccgactacct 60

tggtgatc 68

<210> 19
<211> 68
<212> DNA
<213> Artificial Sequence

<220>
<223> Description of Artificial Sequence: Synthetic DNA

<400> 19
atctgaatca gctcccctgg aatgcagggg agcggcaaga ttaattcccc tgctcgcgca 60

ggctgggt                                                          68

<210> 20
<211> 72
<212> DNA
<213> Artificial Sequence

<220>
<223> Description of Artificial Sequence: Synthetic DNA

<400> 20
tttttagtag cttaaatgtg attcaacatc actggagaaa gtcttatgat gaacaaacat 60

gtaaataaag ta                                                     72

<210> 21
<211> 71
<212> DNA
<213> Artificial Sequence

<220>
<223> Description of Artificial Sequence: Synthetic DNA

<400> 21
ggattatctg aatcagctcc cctggaatgc aggggagcgg caagattagt tgactttttg 60

ttctgcaaaa t                                                      71

## Description

### Technical Field

The present invention relates to a microorganism having an ability to produce lactic acid and a process for producing lactic acid using the microorganism.

### Background Art

Lactic acid-producing microorganisms have been reported and include lactic acid bacteria of the genera *Lactobacillus, Lactococcus,* and the like; filamentous fungi of the genus *Rhizopus;* yeasts of the genus *Saccharomyces* and the like; and *Escherichia coli.* However, it is known that lactic acid bacteria show complex auxotrophy and the optical purity of the lactic acid is low (Non-Patent Documents 1 and 2); that filamentous fungi produce a large amount of by-products such as glycerol, ethanol and fumaric acid (Non-Patent Documents 3 and 4); and even when recombinant strains are used, yeast produce a large amount of ethanol as by-product and the yield with respect to the amount of sugar consumed is low (Non-Patent Document 5).

Known methods for producing lactic acid using *Escherichia coli* include methods using strains defective in the pyruvate formate-lyase gene or genes related to the production of organic acids and ethanol as by-products (Non-Patent Document 6), and methods using mutant strains of the phosphotransacetylase gene or phosphoenolpyruvate carboxylase gene (Non-Patent Document 7); however, the lactic acid productivity is low in all of the above methods.

4-Hydroxybenzoate polyprenyltransferase (hereinafter referred to as UbiA protein) and 2-octaprenylphenol→2-octaprenyl-6-methoxyphenol flavin reductase (hereinafter referred to as UbiB protein) of *Escherichia coli* are proteins involved in the biosynthesis of ubiquinones in *Escherichia coli.* The amino acid sequences of the UbiA protein and UbiB protein and nucleotide sequences of genes encoding these proteins are known (Non-Patent Documents 8 and 9). Moreover, for many microorganisms, the entire nucleotide sequences of these chromosomal DNAs have been elucidated (Non-Patent Document 10).

It is known that an *Escherichia coli* strain with a ubiquinone biosynthetic gene mutation accumulates 2.5 g/L of D-lactic acid (Non-Patent Documents 11 and 12), and that mutant strain (strain AN59) is a mutant strain of the *ubiE* gene (Non-Patent Document 13). Lactic acid productivity in strains that have a decreased activity of other ubiquinone biosynthetic gene products is unknown, and so is the acetic acid production from mutant strains of ubiquinone biosynthetic genes, and the like.

Acetic acid is a by-product of lactic acid fermentation, and interferes with the purification and polymerization of lactic acid. Therefore, inexpensive methods for producing lactic acid that have low production of acetic acid by-product are in demand.
Non-Patent Document 1: Appl. Microbiol. Biotechnol., 45, 307(1996).
Non-Patent Document 2: Enzyme Microb. Technol., 26, 87(2000).
Non-Patent Document 3: Appl. Biochem. Biotechnol., 51, 57(1995).
Non-Patent Document 4: J. Biosci. Bioeng., 97, 19(2004).
Non-Patent Document 5: Appl. Environ. Microbiol., 71, 2789(2005).
Non-Patent Document 6: Appl. Environ. Microbiol., 69, 399(2003).
Non-Patent Document 7: Appl. Environ. Microbiol., 65, 1384(1999).
Non-Patent Document 8: FEBS Letters, 307, 347(1992).
Non-Patent Document 9: Science, 257, 771(1992).
Non-Patent Document 10: http://www.tigr.org/tdb/mdb/mdbcomplete.html
Non-Patent Document 11: J. Bacteriol., 99, 450(1969).
Non-Patent Document 12: Biochem. J., 117, 551(1970).
Non-Patent Document 13: J Bacteriol., 182, 5139(2000).

### Disclosure of the Invention

### [Problems to be Solved by the Invention]

An objective of the present invention is to provide a microorganism having improved lactic acid productivity and a process for producing lactic acid using the microorganism.

### [Means for Solving the Problems]

The present invention relates to the following:
(1) a microorganism, in which the activity of 4-hydroxybenzoate polyprenyltransferase or 2-octaprenylphenol→2-octaprenyl-6-methoxyphenol flavin reductase is reduced or lost, and which has an ability to produce lactic acid;
(2) the microorganism according to (1), which comprises a chromosomal DNA in which a gene encoding a protein having 4-hydroxybenzoate polyprenyltransferase activity or a protein having 2-octaprenylphenol→2-octaprenyl-6-methoxyphenol flavin reductase activity is partially or completely defective;
(3) the microorganism according to (2), wherein the gene encodes a protein comprising the amino acid sequence of SEQ ID NO: 1 or SEQ ID NO: 2; or a protein having a homology of 80% or more with the amino acid sequence of SEQ ID NO: 1 or SEQ ID NO: 2, and having 4-hydroxybenzoate polyprenyltransferase activity or 2-octaprenylphenol→2-octaprenyl-6-methoxyphenol flavin reductase activity;
(4) the microorganism according to (2), wherein the gene is a gene comprising the nucleotide sequence of SEQ ID NO: 3 or 4; or a gene which hybridizes under stringent conditions with a polynucleotide comprising a nucleotide sequence complementary to the nucleotide sequence of SEQ ID NO: 3 or 4, and encodes a protein having 4-hydroxybenzoate polyprenyltransferase activity or 2-octaprenylphenol→2-octaprenyl-6-methoxyphenol flavin reductase activity;
(5) a microorganism according to any one of (1) to (4), wherein the microorganism has an enhanced ability to produce lactic acid by a method selected from the following [1] to [5]:
   [1] a method for partially or completely releasing at least one of the mechanisms that regulate biosynthesis of lactic acid;
   [2] a method for enhancing the expression of at least one of the enzymes involved in biosynthesis of lactic acid;
   [3] a method for increasing the copy number of at least one of the enzyme genes involved in biosynthesis of lactic acid;
   [4] a method for partially or completely blocking at least one of the branched metabolic pathways of lactic acid biosynthesis for metabolites other than useful substances;
   [5] a method for selecting a cell strain that is highly resistant to a lactic acid analogue as compared with a wild type strain;
(6) the microorganism according to any one of (1) to (5), wherein the microorganism belongs to the genus *Erwinia, Serratia, Salmonella, Escherichia, Proteus, Pseudomonas, Acidithiobacillus, Acinetobacter, Agrobacterium, Bacillus, Bordetella, Bradyrhizobium, Brucella, Burkholderia, Caulobacter, Chloroflexus, Clostridium, Coxiella, Desulfitobacterium, Geobacter, Haemophilus, Legionella, Leptospira, Magnetococcus, Magnetospirillum, Mannheimia, Mesorhizobium, Methylobacillus, Methylobacterium, Mycobacterium, Neisseria, Nitrosomonas, Nostoc, Pasteurella, Prochlorococcus, Rhodobacter, Rhodococcus, Rhodopseudomonas, Rickettsia, Shigella, Sinorhizobium, Sphingomonas, Streptomyces, Synechococcus, Synechocystis, Vibrio, Xylella, Yersinia, Zymomonas* or *Xanthomonas;*
(7) the microorganism according to any one of (1) to (6), wherein the microorganism is selected from the group consisting of *Erwinia berbicola, Erwinia amylovora, Erwinia carotovora, Serratia marcescens, Serratia ficaria, Serratia fonticola, Serratia liquefaciens, Salmonella enterica, Salmonella enteritidis, Salmonella paratyphi, Salmonella typhi, Salmonella dublin, Salmonella typhimurium, Escherichia coli, Proteus rettgeri, Pseudomonas putida, Pseudomonas fluorescens, Pseudomonas aeruginosa, Pseudomonas dacunhae, Pseudomonas fluorescens, Pseudomonas syringae, Pseudomonas thazdinophilum, Acidithiobacillus ferrooxidans, Acinetobacter sp.* ATCC 33305, *Agrobacterium tumefaciens, Bacillus halodurans, Bordetella bronchiseptica, Bordetella parapertussis, Bordetella pertussis, Bradyrhizobium japonicum, Brucella melitensis, Burkholderia pseudomallei, Caulobacter crescentus, Chloroflexus aurantiacus, Clostridium acetobutylicum, Clostridium botulinum, Clostridium diffcile, Coxiella burnetii, Desulfitobacter iumhafniense, Geobacter metallireducens, Haemophilus ducreyi, Legionella pneumophila, Leptospira interrogans, Magnetococcus* MC-1, *Magnetospirillum magnetotacticum, Mannheimia haemolytica, Mesorhizobium loti, Methylobacillus flagellatus, Methylobacterium extorquens, Mycobacterium bovis, Mycobacterium leprae, Mycobacterium marinum, Mycobacterium tuberculosis, Neisseria gonorrhoeae, Neisseria meningitidis, Nitrosomonas europaea, Nostoc punctiforme, Pasteurella multocida, Prochlorococcus marinus, Rhodobacter capsulatus, Rhodococcus sp.* strain 124, *Rhodopseudomonas palustris, Rickettsia prowazekii, Shigella flexneri, Sinorhizobium meliloti, Sphingomonas aromaticivorans, Streptomyces avermitilis, Streptomyces coelicolor, Synechococcus sp.* WH8102, *Synechocystis sp.* PCC6803, *Vibrio cholerae, Xylella fastidiosa, Xylella fastidiosa, Yersinia pestis, Zymomonas mobilis, Xanthomonas ovyzae* and *Xanthomonas capestris;* and
(8) a process for producing lactic acid, which comprises culturing the microorganism according to any one of (1) to (7) in a medium so as to produce and accumulate lactic acid in the culture, and recovering lactic acid from the culture.

### [Effects of the Invention]

The present invention provides a microorganism having improved lactic acid productivity and a process for producing lactic acid using the microorganism.

### Best Mode for Carrying Out the Invention

### 1. Microorganisms of the present invention

Microorganisms of the present invention are not particularly limited so long as they have the ability to produce lactic acid, and their 4-hydroxybenzoate polyprenyltransferase activity or 2-octaprenylphenol→2-octaprenyl-6-methoxyphenol flavin reductase activity is reduced or lost. Examples include microorganisms that have an ability to produce lactic acid, and are partially or completely defective in a gene encoding a protein having 4-hydroxybenzoate polyprenyltransferase activity (hereinafter referred to as UbiA protein) or a gene encoding a protein having 2-octaprenylphenol→2-octaprenyl-6-methoxyphenol flavin reductase activity (hereinafter referred to as UbiB protein) in their chromosomal DNA.

In this description, "gene" includes structural genes and regions that have specific regulatory functions such as promoters and operators. Microorganisms having chromosomal DNA in which the entire or part of a gene encoding UbiA protein or UbiB protein is defective include microorganisms, as a result of nucleotide deletion in gene nucleotide sequences in chromosomal DNA, in which: (1) transcriptional regulation of the promoter, operator, or the like of the UbiA protein- or UbiB protein-encoding gene does not function and the UbiA protein or UbiB protein is not expressed; (2) a frameshift occurred and the UbiA protein or UbiB protein is not expressed as an active protein; or (3) the entire or part of a structural gene in the UbiA protein- or UbiB protein-encoding gene is defective. The microorganisms of (3) are preferable, and microorganisms in which an entire structural gene in the gene encoding UbiA protein or UbiB protein is defective are even more preferable.

The phrase "part of a structural gene in the gene encoding UbiA protein or UbiB protein is defective" may refer to a single nucleotide deletion in the structural gene portion, the deletion is preferably a deletion of five to ten nucleotides in the structural gene, more preferably ten to 50 nucleotides, and yet more preferably 50 to 100 nucleotides.

Microorganisms with reduced 4-hydroxybenzoate polyprenyltransferase or 2-octaprenylphenol→2-octaprenyl-6-methoxyphenol flavin reductase activity are not particularly limited as long as the activity is reduced as compared with microorganisms having a gene encoding the wild type UbiA protein or UbiB protein. However, generally, they are microorganisms in which the activity is reduced to 60% or less, preferably 40%, more preferably 20% or less, yet more preferably 10% or less, and particularly preferably 5% or less.

The gene encoding UbiA protein or UbiB protein is not particularly limited as long as the gene encodes a protein having 4-hydroxybenzoate polyprenyltransferase activity or a protein having 2-octaprenylphenol→2-octaprenyl-6-methoxyphenol flavin reductase activity. However, examples include: genes encoding a protein comprising the amino acid sequence shown in SEQ ID NO: 1 or 2; genes encoding a protein having a homology of 80% or more, preferably 90% or more, more preferably 95% or more, yet more preferably 98% or more, and particularly preferably 99% or more with a protein comprising the amino acid sequence shown in SEQ ID NO: 1 or 2 and also having 4-hydroxybenzoate polyprenyltransferase activity or 2-octaprenylphenol→2-octaprenyl-6-methoxyphenol flavin reductase activity; genes comprising the nucleotide sequence shown in SEQ ID NO: 3 or 4, which are genes encoding a protein comprising the amino acid sequence shown in SEQ ID NO: 1 or 2, respectively; and genes which hybridize under stringent conditions with a polynucleotide comprising a nucleotide sequence that is complementary to the nucleotide sequence shown in SEQ ID NO: 3 or 4 and which encode a protein having 4-hydroxybenzoate polyprenyltransferase activity or 2-octaprenylphenol→2-octaprenyl-6-methoxyphenol flavin reductase activity.

The homology of amino acid sequences and nucleotide sequences can be determined by using the BLAST algorithm by Karlin and Altschul [Pro. Natl. Acad. Sci. USA, 90, 5873 (1993)] or FASTA [Methods Enzymol., 183, 63 (1990)]. Based on this BLAST algorithm, programs called BLASTN and BLASTX have been developed [J. Mol. Biol., 215, 403(1990)]. When a nucleotide sequence is analyzed using BLASTN based on BLAST, the parameters are set for example as follows: score=100 and word length=12. When an amino acid sequence is analyzed using BLASTX based on BLAST, the parameters are set, for example, as follows: score=50 and word length=3. When the BLAST and Gapped BLAST programs are used, default parameters of each program are used. The specific procedures for these analytical methods are known (http://www.ncbi.nlm.nih.gov.).

The term "hybridize" as used herein indicates that a gene hybridizes with a polynucleotide comprising a specific nucleotide sequence or a part thereof. Therefore, the polynucleotide comprising a specific nucleotide sequence or a part thereof is a polynucleotide that can be used as a probe in northern or Southern blot analysis, or as an oligonucleotide primer in PCR analysis. Polynucleotides that are used as probes include polynucleotides comprising at least 100 nucleotides or more, preferably 200 nucleotides or more, and more preferably 500 nucleotides or more, while polynucleotides that are used as primers include polynucleotides comprising at least ten nucleotides or more, and preferably 15 nucleotides or more.

Methods for experiments of polynucleotide hybridization are well known. For example, those skilled in the art can determine the hybridization conditions following the description of the present application. Hybridization conditions are described in Molecular Cloning, 2nd edition and 3rd edition (2001); Methods for General and Molecular Bacteriology, ASM Press (1994); Immunology Methods Manual, Academic Press (Molecular); and so forth, and many other standard textbooks can be followed.

Preferably, the above "stringent hybridization condition" is an overnight incubation at 42°C of a polynucleotide, preferably a DNA-immobilized filter, with a probe, preferably a DNA probe, in a solution containing 50% formamide, 5x SSC (750 mM sodium chloride and 75 mM sodium citrate), 50 mM sodium phosphate (pH 7.6), 5x Denhardt's solution, 10% dextran sulfate and 20 µg/l denatured salmon sperm DNA, followed by washing the filter in, for example, a 0.2x SSC solution at about 65°C. However, conditions of lower stringency can also be used. Modifications of the stringency conditions can be accomplished by adjusting the formamide concentration (the lower the formamide concentration, the lower the stringency) and changing the salt concentration or temperature condition. Examples of a low stringency condition include an overnight incubation at 37°C in a solution containing 6x SSCE (20x SSCE refers to 3 mol/l sodium chloride, 0.2 mol/l sodium dihydrogenphosphate and 0.02 mol/l EDTA, pH 7.4), 0.5% SDS, 30% formamide, 100 µg/l denatured salmon sperm DNA, followed by washing with a 1x SSC, 0.1 % SDS solution at 50°C. Moreover, an example of a condition of even lower stringency is a condition in which a solution of high salt concentration (for example, 5x SSC) is used for hybridization followed by washing in the above-described low stringency condition.

The various conditions described above can also be set by adding or changing blocking reagents which are used to suppress the background in hybridization experiments. The aforementioned addition of blocking reagents may involve a change in the hybridization conditions to adjust the conditions.

Examples of genes that can be hybridized under the aforementioned stringent conditions include genes having a homology of at least 90% or more, preferably 95% or more, more preferably 97% or more, yet more preferably 98% or more, and particularly preferably 99% or more with the nucleotide sequence shown in SEQ ID NO: 3 or 4, as calculated using, for example, BLAST and FASTA described above based on, for example, the parameters described above.

Whether a gene hybridizing under stringent conditions with a polynucleotide comprising a complementary nucleotide sequence of the nucleotide sequence shown in SEQ ID NO: 3 or 4 is a gene encoding a protein having 4-hydroxybenzoate polyprenyltransferase activity or 2-octaprenylphenol→2-octaprenyl-6-methoxyphenol flavin reductase activity can be confirmed, for example, by generating proteins encoded by the gene using genetic recombination methods and measuring protein activity.

Moreover, the microorganisms of the present invention are preferably prokaryotes and more preferably bacteria.

Examples of bacteria include microorganisms belonging to the following genera: *Erwinia, Serratia, Salmonella, Escherichia, Proteus, Pseudomonas, Acidithiobacillus, Acinetobacter, Agrobacterium, Bacillus, Bordetella, Bradyrhizobium, Brucella, Burkholderia, Caulobacter, Chloroflexus, Clostridium, Coxiella, Desulfitobacterium, Geobacter, Haemophilus, Legionella, Leptospira, Magnetococcus, Magnetospirillum, Mannheimia, Mesorhizobium, Methylobacillus, Methylobacterium, Mycobacterium, Neisseria, Nitrosomonas, Nostoc, Pasteurella, Prochlorococcus, Rhodobacter, Rhodococcus, Rhodopseudomonas, Rickettsia, Shigella, Sinorhizobium, Sphingomonas, Streptomyces, Synechococcus, Synechocystis, Vibrio, Xylella, Yersinia, Zymomonas* and *Xanthomonas.*

Moreover, examples of bacteria belonging to the above genera include *Erwinia berbicola, Erwinia amylovora, Erwinia carotovora, Serratia marcescens, Serratia ficaria, Serratia fonticola, Serratia liquefaciens, Salmonella enterica, Salmonella enteritidis, Salmonella paratyphi, Salmonella typhi, Salmonella dublin, Salmonella typhimurium, Escherichia coli, Proteus rettgeri, Pseudomonas putida, Pseudomonas fluorescens, Pseudomonas aeruginosa, Pseudomonas dacunhae, Pseudomonas fluorescens, Pseudomonas syringae, Pseudomonas thazdinophilum, Acidithiobacillus ferrooxidans, Acinetobacter sp.* ATCC 33305, *Agrobacterium tumefaciens, Bacillus halodurans, Bordetella bronchiseptica, Bordetella parapertussis, Bordetella pertussis, Bradyrhizobium japonicum, Brucella melitensis, Burkholderia pseudomallei, Caulobacter crescentus, Chloroflexus aurantiacus, Clostridium acetobutylicum, Clostridium botulinum, Clostridium difficile, Coxiella burnetii, Desulfitobacter iumhafniense, Geobacter metallireducens, Haemophilus ducreyi, Legionella pneumophila, Leptospira interrogans, Magnetococcus* MC-1, *Magnetospirillum magnetotacticum, Mannheimia haemolytica, Mesorhizobium loti, Methylobacillus flagellatus, Methylobacterium extorquens, Mycobacterium bovis, Mycobacterium leprae, Mycobacterium marinum, Mycobacterium tuberculosis, Neisseria gonorrhoeae, Neisseria meningitidis, Nitrosomonas europaea, Nostoc punctiforme, Pasteurella multocida, Prochlorococcus marinus, Rhodobacter capsulatus, Rhodococcus sp.* strain 124, *Rhodopseudomonas palustris, Rickettsia prowazekii, Shigella flexneri, Sinorhizobium meliloti, Sphingomonas aromaticivorans, Streptomyces avermitilis, Streptomyces coelicolor, Synechococcus sp.* WH8102, *Synechocystis sp.* PCC6803, *Vibrio cholerae, Xylella fastidiosa, Xylella fastidiosa, Yersinia pestis, Zymomonas mobilis, Xanthomonas ovyzae* and *Xanthomonas capestris.* A more preferred example is *E*. *coli.*

### 2. Methods for constructing the microorganism of the present invention

Microorganisms of the present invention can be constructed by: (1) a method for deleting the entire or part of a gene encoding UbiA protein or UbiB protein in chromosomal DNA of a microorganism that has the ability to produce lactic acid; or (2) a method that imparts the ability to produce lactic acid to microorganisms that have the entire or part of a gene encoding UbiA protein or UbiB protein deleted from their chromosomal DNA.

The microorganisms having the ability to produce lactic acid are not particularly limited as long as the microorganisms have this ability. Examples of such microorganisms include naturally isolated strains that have the ability, and microorganisms that have been artificially imparted with the ability to produce lactic acid by known methods.

These known methods include the following methods, and these methods can be used alone or in combination:
(a) a method for partially or completely releasing at least one of the mechanisms that regulate the biosynthesis of lactic acid;
(b) a method for enhancing the expression of at least one of the enzymes that are involved in the biosynthesis of lactic acid;
(c) a method for increasing the copy number of at least one of the genes for enzymes involved in the biosynthesis of lactic acid;
(d) a method for partially or completely blocking at least one of the branching metabolic pathways of the lactic acid biosynthesis pathway that produce metabolites other than a useful substance;
(e) a method for selecting a cell strain that is highly resistant to a lactic acid analogue as compared with wild type strains.

Examples of method (a) above include a method that releases the regulation of lactic acid biosynthesis using the pyruvate formate-lyase gene [Appl. Environ. Microbiol., 69, 399 (2003)]. Examples of method (c) include a method that introduces six copies of a DNA encoding an L-lactic acid dehydrogenase into chromosomal DNA [Appl. Environ. Microbiol., 71, 2789 (2005)]. Examples of method (d) include a method that introduces mutations into the phosphotransacetylase gene or phosphoenolpyruvate carboxylase gene [Appl. Environ. Microbiol., 65, 1384 (1999)].

Methods for obtaining microorganisms that have the entire or part of a gene encoding UbiA protein or UbiB protein deleted from their chromosomal DNA are not limited, as long as the microorganisms can be obtained by the methods. The microorganisms can be obtained, for example, by introducing deletion, substitution, and addition of nucleotides into a gene encoding UbiA protein or UbiB protein in a microbial chromosomal DNA using the following nucleotide sequence information of UbiA protein- or UbiB protein-encoding genes in chromosomal DNA of microorganisms. Nucleotide sequences of genes encoding UbiA protein or UbiB protein of a microorganism can be determined by searching various DNA sequence databases using the nucleotide sequence shown in SEQ ID NO: 3 or 4 as a query, and identifying and obtaining UbiA protein- or UbiB protein-encoding genes by Southern hybridization of chromosomal DNAs of various microorganisms using the entire or part of a polynucleotide comprising a complementary nucleotide sequence of the nucleotide sequence shown in SEQ ID NO: 3 or 4 as probe. Alternatively, nucleotide sequences can be determined by identifying and obtaining UbiA protein- or UbiB protein-encoding genes by PCR using primers designed based on the nucleotide sequence shown in SEQ ID NO: 3 or 4, using chromosomal DNAs of various microorganisms as template, and then analyzing the nucleotide sequences of the genes by conventional methods. The chromosomal DNA used in the Southern hybridization or PCR may be the chromosomal DNA of any microorganism. The chromosomal DNA is preferably that from a microorganism belonging to the following genera: *Erwinia, Serratia, Salmonella, Escherichia, Proteus, Pseudomonas, Acidithiobacillus, Acinetobacter, Agrobacterium, Bacillus, Bordetella, Bradyrhizobium, Brucella, Burkholderia, Caulobacter, Chloroflexus, Clostridium, Coxiella, Desulfitobacterium, Geobacter, Haemophilus, Legionella, Leptospira, Magnetococcus, Magnetospirillum, Mannheimia, Mesorhizobium, Methylobacillus, Methylobacterium, Mycobacterium, Neisseria, Nitrosomonas, Nostoc, Pasteurella, Prochlorococcus, Rhodobacter, Rhodococcus, Rhodopseudomonas, Rickettsia, Shigella, Sinorhizobium, Sphingomonas, Streptomyces, Synechococcus, Synechocystis, Vibrio, Xylella, Yersinia, Zymomonas* and *Xanthomonas.* More preferably, the chromosomal DNA is that of: *Erwinia berbicola, Erwinia amylovora, Erwinia carotovora, Serratia marcescens, Serratia ficaria, Serratia fonticola, Serratia liquefaciens, Salmonella enterica, Salmonella enteritidis, Salmonella paratyphi, Salmonella typhi, Salmonella dublin, Salmonella typhimurium, Escherichia coli, Proteus rettgeri, Pseudomonas putida, Pseudomonas fluorescens, Pseudomonas aeruginosa, Pseudomonas dacunhae, Pseudomonas fluorescens, Pseudomonas syringae, Pseudomonas thazdinophilum, Acidithiobacillus ferrooxidans, Acinetobacter sp.* ATCC 33305, *Agrobacterium tumefaciens, Bacillus halodurans, Bordetella bronchiseptica, Bordetella parapertussis, Bordetella pertussis, Bradyrhizobium japonicum, Brucella melitensis, Burkholderia pseudomallei, Caulobacter crescentus, Chloroflexus aurantiacus, Clostridium acetobutylicum, Clostridium botulinum, Clostridium difficile, Coxiella burnetii, Desulfitobacter iumhafniense, Geobacter metallireducens, Haemophilus ducreyi, Legionella pneumophila, Leptospira interrogans, Magnetococcus* MC-1, *Magnetospirillum magnetotacticum, Mannheimia haemolytica, Mesorhizobium loti, Methylobacillus flagellatus, Methylobacterium extorquens, Mycobacterium bovis, Mycobacterium leprae, Mycobacterium marinum, Mycobacterium tuberculosis, Neisseria gonorrhoeae, Neisseria meningitidis, Nitrosomonas europaea, Nostoc punctiforme, Pasteurella multocida, Prochlorococcus marinus, Rhodobacter capsulatus, Rhodococcus sp.* strain 124, *Rhodopseudomonas palustris, Rickettsia prowazekii, Shigella flexneri, Sinorhizobium meliloti, Sphingomonas aromaticivorans, Streptomyces avermitilis, Streptomyces coelicolor, Synechococcus sp.* WH8102, *Synechocystis sp.* PCC6803, *Vibrio cholerae, Xylella fastidiosa, Xylella fastidiosa, Yersinia pestis, Zymomonas mobilis, Xanthomonas ovyzae* and *Xanthomonas capestris.*

Hybridization and PCR can be carried out following routine methods, such as methods described in Molecular Cloning, 2nd edition and 3rd edition (2001); Methods for General and Molecular Bacteriology, ASM Press (1994), or many other standard textbooks can be followed.

Examples of a gene encoding UbiA protein or UbiB protein include: genes encoding a protein comprising the amino acid sequence shown in SEQ ID NO: 1 or 2; genes comprising the nucleotide sequence shown in SEQ ID NO: 3 or 4; *Shigella flexneri* 2a-derived *ubiA* gene and *yigR* gene (Genbank accession no. AE005674); and *Salmonella enterica-derived ubiA* gene and *aarF* gene (Genbank accession no. NC004631).

Examples of a method for introducing a deletion, substitution, or addition of nucleotides into a gene in a chromosomal DNA of a microorganism include methods utilizing homologous recombination. Examples of a general method utilizing homologous recombination include methods using a plasmid for homologous recombination constructed by linking a mutant gene into which deletion, substitution, or addition of a nucleotide has been introduced, with a plasmid DNA comprising a drug resistance gene and which cannot autonomously replicate in the host cell into which deletion, or the like of a nucleotide is to be introduced.

The plasmid for homologous recombination is introduced into the host cell by a routine method. Then, transformed strains whose chromosomal DNA has been incorporated by homologous recombination with the plasmid for homologous recombination are selected using a drug resistance marker. The obtained transformed strains are cultured in a medium without the drug for several hours to one day, and are then spread on an agar medium containing the drug and on an agar medium without the drug. By selecting strains that do not grow on the former medium but can grow on the latter medium, strains whose chromosomal DNA has undergone the second homologous recombination can be obtained. Introduction of a nucleotide deletion, substitution, or addition into a gene of interest in chromosomal DNA can be confirmed by determining the nucleotide sequence of the region in the chromosomal DNA at the location of the gene into which the deletion or the like has been introduced.

Examples of microorganisms in which deletions, substitutions, or additions of a nucleotide can be introduced into a gene of interest in chromosomal DNA by the above method include microorganisms belonging to the genus *Escherichia.*

Further, examples of an efficient method for introducing deletions, substitutions, or additions of a nucleotide at multiple genes using homologous recombination include methods using linear DNAs.

Specifically, in this method, linear DNAs containing a gene into which deletion, substitution, or addition of a nucleotide is to be introduced is incorporated into cells to cause homologous recombination between the chromosomal DNA and the introduced linear DNAs. This method is applicable to any microorganism, so long as the microorganism efficiently incorporates linear DNAs. The microorganisms are preferably *Escherichia,* more preferably *Escherichia coli,* and yet more preferably *Escherichia coli* expressing λ phage-derived groups of recombinant proteins (Red recombination system).

An example of *Escherichia coli* expressing the λ Red recombination system is the *Escherichia coli* JM101 strain carrying pKD46, which is a plasmid DNA comprising genes for the λ Red recombination system (available from *Escherichia coli* Genetic Stock Center, Yale University, U.S.A.).

Examples of DNAs used for homologous recombination include the following:
(a) linear DNAs comprising, at both ends of the drug resistance gene, DNAs that exist on both sides outside of the region in chromosomal DNA to be introduced with nucleotide deletion, substitution, or addition, or DNAs that are homologous to these DNAs;
(b) linear DNAs in which DNAs existing on both sides outside of the region in chromosomal DNA to be introduced with nucleotide deletion, substitution or addition, or DNAs that are homologous to these DNAs, are directly linked;
(c) linear DNAs comprising on both sides of a DNA in which a drug resistance gene and a gene that can be used for negative selection are linked, DNAs existing on both sides outside of the region in chromosomal DNA to be introduced with nucleotide deletion, substitution or addition, or DNAs that are homologous to these DNAs; and
(d) DNAs further comprising, in the linear DNAs of (a) above, a nucleotide sequence recognized by the yeast-derived Flp recombinase [Proc. Natl. Acad. Sci. USA., 82, 5875 (1985)] between the drug resistance gene and the DNAs existing on both sides outside of the region in chromosomal DNA.

Any drug resistance gene can be used as long as the drug resistance gene imparts resistance towards a drug to which the host microorganism shows sensitivity. When *Escherichia coli* is used as the host microorganism, examples of drug resistance genes include the kanamycin resistance gene, chloramphenicol resistance gene, gentamicin resistance gene, spectinomycin resistance gene, tetracycline resistance gene and ampicillin resistance gene.

The "gene that can be used for negative selection" refers to a gene that is lethal under certain culture conditions to a host microorganism when the gene is expressed in the microorganism. Examples of such genes include the *sacB* gene derived from microorganisms belonging to the genus *Bacillus* [Appl. Environ. Microbiol., 59, 1361-1366 (1993)] and the *rpsL* gene derived from microorganisms belonging to the genus *Escherichia* [Genomics, 72, 99-104 (2001)].

DNAs that are homologous to the DNAs located on both sides outside of the region in chromosomal DNA to be introduced with a substitution or deletion, and which exist on both ends of the above linear DNAs, are placed in the linear DNAs in the same direction as the chromosomal DNA, and their length is preferably about 10 bp to 100 bp, more preferably about 20 bp to 50 bp, and further preferably about 30 bp to 40 bp.

The nucleotide sequence recognized by the yeast-derived Flp recombinase is not particularly limited, so long as the protein recognizes the nucleotide sequence and catalyzes homologous recombination. Preferred examples are DNAs comprising the nucleotide sequence shown in SEQ ID NO: 13; and DNAs comprising a nucleotide sequence with a deletion, substitution, or addition of one to several nucleotides in these DNAs and in which the nucleotide sequence is recognized by the yeast-derived Flp recombinase and catalyzes homologous recombination.

The term "homologous" indicates that the above linear DNAs have a degree of homology such that homologous recombination occurs in a region of interest in the chromosomal DNA. Specifically, examples include a homology of 80% or more, preferably 90% or more, more preferably 95% or more, and further preferably 100%.

The homology of the aforementioned nucleotide sequences can be determined using programs such as BLAST and FASTA described above.

The above-described linear DNAs can be produced by PCR. The linear DNAs of interest can also be obtained by constructing DNAs comprising the above linear DNAs on a plasmid, followed by restriction enzyme treatment.

Methods for introducing deletion, substitution, or addition of a nucleotide into the chromosomal DNA of a microorganism include methods 1 to 4 below:
Method 1: a method that introduces the linear DNAs of the above (a) or (d) into a host microorganism and uses a drug resistance marker to select for transformed strains whose chromosomal DNA has been inserted with the linear DNAs through homologous recombination.
Method 2: a method that introduces the linear DNAs of the above (b) into a transformed strain obtained by the above Method 1 and substitutes or deletes the region in the chromosomal DNA of the microorganism by deleting the drug resistance gene inserted in the chromosomal DNA by Method 1.
Method 3: a method that:
   [1] introduces the linear DNAs of the above (c) into a host microorganism, and uses a drug resistance marker to select for transformed strains whose chromosomal DNA has been inserted with the linear DNAs through homologous recombination;
   [2] synthesizes DNAs in which a DNA homologous to the DNA existing on both sides outside of the region in chromosomal DNA to be introduced with a substitution or deletion is linked in the same direction as the chromosomal DNA, and introduces these DNAs into the transformed strains obtained in [1] above; and
   [3] cultures the transformed strains from procedure [2] above under conditions in which a negative selection gene is expressed, and selects strains that can grow in this culture as strains in which the drug resistance gene and the negative selection gene have been deleted from the chromosomal DNA.
Method 4: a method that:
   [1] introduces the linear DNAs of the above (d) into a host microorganism and uses a drug resistance marker to select for transformed strains whose chromosomal DNA has been inserted with the linear DNAs through homologous recombination; and
   [2] introduces an Flp recombinase gene expression plasmid into the transformed strains obtained in [1], expresses the gene, and obtains strains that are sensitive to the drug used in [1] above.

In the above methods, any method can be used for introducing linear DNAs into a host microorganism, as long as the method introduces DNAs into the microorganism. Examples include methods using calcium ions [Proc. Natl. Acad. Sci. USA, 69, 2110 (1972)], the protoplast method (Japanese Published Unexamined Patent Application No. S63-2483942), and the electroporation method [Nucleic Acids Res., 16, 6127 (1988)].

For the linear DNA used in Method 2 or Method 3 [2], a linear DNA in which an arbitrary gene to be inserted into chromosomal DNA is incorporated in the vicinity of the center of the DNA can be used, so that the drug resistance gene can be deleted and the arbitrary gene can be simultaneously inserted into the chromosomal DNA.

The above Methods 2 to 4 are methods that leave no foreign genes, such as the drug resistance gene and the negative selection gene, on the chromosomal DNA of the transformed strains obtained at the end. Therefore, by using these methods, it is possible to readily produce microorganisms with nucleotide deletions, substitutions, or additions in two or more different regions of the chromosomal DNA by repeating these methods using the same drug resistance gene and negative selection gene.

It is possible to confirm that the activity of 4-hydroxybenzoate-polyprenyltransferase or 2-octaprenylphenol→2-octaprenyl-6-methoxyphenol flavin reductase is reduced or lost in a mutant strain obtained by the above methods by, for example, measuring and comparing ubiquinone productivities of the mutant strain and the original strain using known methods.

Moreover, in the present invention, microorganisms having a high L-lactic acid productivity can be constructed by reducing or abolishing the activity of D-lactic acid dehydrogenase (EC 1.1.1.28) in a microorganism, and more preferably, by enhancing the activity of L-lactic acid dehydrogenase (EC 1.1.2.27). Conversely, microorganisms having a high D-lactic acid productivity can be generated by reducing or abolishing L-lactic acid dehydrogenase activity, and more preferably, by enhancing D-lactic acid dehydrogenase activity.

The amino acid sequences of D-lactic acid dehydrogenase and L-lactic acid dehydrogenase, and the nucleotide sequences of genes encoding these enzymes are known for many microorganisms. The sequences can be readily obtained from various databases.

Accordingly, microorganisms in which the activity of D-lactic acid dehydrogenase or L-lactic acid dehydrogenase is reduced or lost can be obtained by deleting the entire or part of the genes for these enzymes in chromosomal DNA, using the obtained sequence information and the aforementioned methods.

Moreover, microorganisms in which the activity of D-lactic acid dehydrogenase or L-lactic acid dehydrogenase is enhanced can be obtained by introducing genes for these enzymes into a microorganism using known methods. Such genes may be incorporated into chromosomal DNA, or may be present as plasmid DNA outside chromosome.

Methods for generating microorganisms with improved L-lactic acid productivity using *Escherichia coli* as the microorganism include a method in which the entire ORF of the *Escherichia coli* D-lactic acid dehydrogenase gene is deleted and a L-lactic acid dehydrogenase gene, preferably the L-lactic acid dehydrogenase gene of *Bacillus subtilis,* is incorporated into the chromosomal DNA.

### 3. Process for producing lactic acid using the microorganism of the present invention

Both D-lactic acid and L-lactic acid can be produced according to the methods of the present invention.

Lactic acid can be produced by culturing in a medium a microorganism of the present invention described in 2 above so as to produce and accumulate lactic acid in a culture and recovering lactic acid from the culture.

The microorganism can be cultured in a medium using standard methods for culturing microorganisms.

Thus, both natural and synthetic media can be used as long as the medium contains carbon sources, nitrogen sources, inorganic salts, and the like that can be assimilated by the microorganism, and allows efficient culturing of the microorganism.

Any carbon sources that can be assimilated by the microorganism can be used. Carbohydrates such as glucose, fructose, sucrose and molasses containing these carbohydrates, starch and starch hydrolysates; organic acids such as acetic acid and propionic acid; alcohols such as ethanol and propanol; and the like can be used.

For nitrogen sources, ammonia; ammonium salts of inorganic or organic acids such as ammonium chloride, ammonium sulfate, ammonium acetate and ammonium phosphate; other nitrogen-containing compounds; peptone; meat extract; yeast extract; corn steep liquor; casein hydrolysates; soybean cake; and soybean cake hydrolysates; various fermentation microorganisms and digests thereof can be used.

For inorganic salts, monopotassium phosphate, dipotassium phosphate, magnesium phosphate, magnesium sulfate, sodium chloride, ferrous sulfate, manganese sulfate, copper sulfate, calcium carbonate and the like can be used.

Culturing is generally carried out under aerobic conditions, for example, by shake culture or submerged culture with aeration and agitation. The culture temperature is preferably 15°C to 40°C, and the culture period is normally five hours to seven days. The pH is kept at 3.0 to 9.0 during culturing. The pH is adjusted with an inorganic or organic acid, an alkaline solution, urea, calcium carbonate, ammonia, or the like.

Lactic acid that is produced and accumulated in the culture can be recovered by a standard method using activated carbon or an ion-exchange resin, an electrodialysis method using an ion-exchange membrane, a method of esterification, distillation and hydrolysis, an extraction method using organic solvents, a method of vacuum distillation and crystallization, or a chromatography method using high-performance liquid chromatography or the like.

Herein below, the present invention will be described with reference to Examples, but it is not to be construed as being limited thereto.

### [Example 1]

### Construction of strains in which a gene encoding UbiA protein or UbiB protein is deleted

PCR was carried out using chromosomal DNA of the *E*. *coli* KM22 strain [Gene, 246, 321-330 (2000)] as template and as the primer set, DNAs comprising the nucleotide sequence shown in SEQ ID NO: 5 or 6, which were designed so that 25 nucleotides of their 3'-ends hybridize with both ends of the kanamycin resistance gene in the chromosomal DNA of the KM22 strain. PCR was carried out using LA-Taq. A 25 µl reaction solution containing 10 ng of chromosomal DNA fragments and 20 pmol each of the primer DNAs was prepared according to the enclosed LA-Taq instructions, and PCR was carried out with the following conditions: heating at 94°C for 2 minutes, followed by 30 cycles of 94°C for 15 seconds, 55°C for 20 seconds and 68°C for 1 minute, and subsequent heating at 72°C for 10 minutes.

Next, DNA fragments in which the nucleotide sequence shown in SEQ ID NO: 7 is added to the upstream of the 5'-end of the kanamycin resistance gene and the nucleotide sequence shown in SEQ ID NO: 8 is added to the downstream of the 3'-end of the kanamycin resistance gene were amplified by PCR by a similar method as described above.

DNA fragments for deleting the gene encoding a UbiA protein (hereinafter referred to as *ubiA* gene) and DNA fragments for deleting the gene encoding a UbiB protein (hereinafter referred to as *ubiB* gene) were prepared as follows. PCR was carried out using the DNA fragments obtained above as template and using, as primer set, DNAs comprising the nucleotide sequence shown in SEQ ID NO: 9 or 10, or DNAs comprising the nucleotide sequence shown in SEQ ID NO: 11 or 12. The DNA comprising the nucleotide sequence shown in SEQ ID NO: 9 or 11 comprises, at its 5'end, a nucleotide sequence comprising 45 nucleotides having a sequence homologous to the vicinity of the 5'-end region of the *ubiA* gene or *ubiB* gene, respectively, and at its 3'end, the nucleotide sequence shown in SEQ ID NO: 7. The DNA comprising the nucleotide sequence shown in SEQ ID NO: 10 or 12 comprises at its 5'end, a nucleotide sequence comprising 45 nucleotides having a sequence homologous to the vicinity of the 3'-end region of the *ubiA* gene or *ubiB* gene, respectively, and at its 3'-end, DNA comprising 25 nucleotides which hybridizes with the nucleotide sequence shown in SEQ ID NO: 8.

PCR was carried out using EX-Taq (Takara Bio Inc.). A 50 µl reaction solution containing 10 ng of the amplified DNA fragments and 10 pmol each of the primer DNAs was prepared according to the enclosed EX-Taq instructions, and PCR was carried out with the following conditions: heating at 94°C for 1 minute, followed by 30 cycles of 94°C for 30 seconds, 55°C for 30 seconds and 72°C for 1 minute, and subsequent cooling at 4°C.

Amplification of DNA fragments comprising a kanamycin resistance gene (km gene) having, at both ends, regions that are homologous to the 45 nucleotides at each end of the genes was confirmed. The DNA fragments were subjected to agarose gel electrophoresis, and then excised from the gel, and purified.

Next, competent cells of the *E. coli* BW25113 strain [Proc. Natl. Acad. Sci., U S A., 97, 6640-6645 (2000)] carrying the pKD46 plasmid, which is capable of expressing the λ Red recombinase, were prepared according to the method described in Gene, 246, 321-330 (2000). Transformation was carried out using 100ng of each of the DNA fragments. Transformation was carried out by electroporation in a 0.1 cm cuvette (BioRad) under the conditions of 1.8 kV and 25 µF. The transformed cells were cultured using SOC medium [20 g/l Bacto tryptone (Difco), 5 g/l Bacto yeast extract (Difco), 0.5 g/l sodium chloride, 0.2 ml/l of 5 mol/l NaOH, 10 ml/l of 1 mol/l magnesium chloride, 10 ml/l of 1 mol/l magnesium sulfate and 10 ml/l of 2M glucose] at 30°C for three hours. Then, the culture solution was spread on an LB agar plate containing 50 µg/ml kanamycin and 10 g/l glucose, and this was incubated at 30°C overnight.

The strains that grew were confirmed to lack the *ubiA* gene or *ubiB* gene in the chromosomal DNA of the *E*. *coli* BW25113 strain and were designated as the *E*. *coli* BW25113ΔubiA strain and *E. coli* BW25113ΔubiB strain, respectively.

### [Example 2]

### Production of lactic acid using the ubiA gene-defective strain or the ubiB gene-defective strain (1)

The *E. coli* BW25113ΔubiA strain, *E. coli* BW25113ΔubiB strain, as well as the *ubiE* gene-defective strain (*E*. *coli* BW25113ΔubiE), *ubiG* gene-defective strain (*E. coli* BW25113ΔubiG), *ubiH* gene-defective strain (*E*. *coli* BW25113ΔubiH) generated according to the method described in Example 1, and the *E*. *coli* BW25113 strain, which were used as controls, were each inoculated into 5 ml of an LB liquid medium containing 10 g/l glucose in a test tube and cultured at 37°C for 17 hours with shaking. 60 µl of the culture was inoculated into 6 ml of a M9 liquid medium (4% glucose, 11.28 g/l M9 Minimal Salts (X5) (Difco), 100 µmol/l calcium chloride, 2 mmol/l magnesium sulfate and 10 µg/ml iron sulfate) containing 3% calcium carbonate and 2% casamino acid in a large test tube and cultured at 37°C for 28 hours with shaking. The cultures were centrifuged after 28 hours. Their supernatants were diluted 10- to 30-fold, and the amounts of D-lactic acid and acetic acid accumulated in the culture solutions were measured using F-kit D-/L-lactic acid, F-kit acetic acid, and F-kit glucose from Roche Diagnostics. The results are shown in Table 1.

**Table 1**

| BACTERIAL STAIN NAMES | D-LACTIC ACID (g/l) | ACETIC ACID (g/l) |
|---|---|---|
| E. coli BW25113 | 0 | n. d. |
| E. coli BW25113ΔubiA | 35.0 | 0.8 |
| E. coli BW25113ΔubiB | 35. 7 | 0.4 |
| E. coli BW25113ΔubiE | 25.0 | 5.0 |
| E. coli BW25113ΔubiG | 28.8 | 4. 1 |
| E. coli BW25113ΔubiH | 29.0 | 6.0 |

| | | |
|---|---|---|
| "n. d." in table 1 means "not determined" | | |

As shown in Table 1, it was revealed that as compared with other ubiquinone biosynthesis gene-defective strains, the *E. coli* BW25113ΔubiA strain and the *E. coli* BW25113ΔubiB strain have high D-lactic acid productivity and low by-production of acetic acid, which is an impurity during lactic acid purification and inhibits the polymerization of lactic acid. Moreover, the optical purity of D-lactic acid was 99% or more.

### [Example 3]

### Production of lactic acid using the ubiA gene-defective strain or the ubiB gene-defective strain (2)

The *E*. *coli* BW25113ΔubiA strain and *E. coli* BW25113ΔubiB strain were each inoculated into 5 ml of an LB liquid medium containing 10 g/l glucose in a test tube and cultured at 37°C for 17 hours with shaking to obtain cultures. 55 µl of each of the cultures was inoculated into 5 ml of a M9 liquid medium (5% glucose, 11.28 g/l M9 Minimal Salts (X5) (Difco), 100 µmol/l calcium chloride, 2 mM magnesium sulfate and 10 µg/ml iron sulfate) containing 6% calcium carbonate and 2% casamino acid in a test tube and cultured at 37°C for 25 hours with shaking. After 25 hours, 1041 µl of 30% glucose, 104 µl of 22.56 g/l M9 Minimal Salts (X10) (Difco), 104 µl of 10% casamino acid, 2 µl of 1 mol/l magnesium sulfate, 1 µl of 10 mg/ml iron sulfate and 0.06 g of calcium carbonate was added to the culture, and culturing was continued. At the 48^{th} hour of culturing, the cultures were recovered and centrifuged. Resulting supernatants were diluted 10- to 1000-fold, and the amounts of D-lactic acid and acetic acid that have accumulated in the culture solutions as well as the amount of remaining sugar were measured using F-kit D-/L-lactic acid, F-kit acetic acid, and F-kit glucose from Roche Diagnostics. The results are shown in Table 2.

**Table 2**

| BACTERIAL STRAIN NAMES | D-LACTIC ACID (g/l) | ACETIC ACID (g/l) | AMOUNT OF RESIDUAL SUGAR (g/l) |
|---|---|---|---|
| E. coli BW25113ΔubiA | 99.6 | 2.0 | 0.2 |
| E. coli BW25113ΔubiB | 100.9 | 1.4 | 0.1 |

As shown in Table 2, *E*. *coli* BW25113ΔubiA strain and *E*. *coli* BW25113ΔubiB strain have the ability to produce as much as about 100 g/l D-lactic acid; however, the amount of acetic acid by-product was significantly low. Moreover, the optical purity of D-lactic acid was 99% or higher.

### [Example 4]

### Construction of microorganism having an ability to produce L-lactic acid

### (1) Generation of E. coli in which the D-lactic acid dehydrogenase gene is defective

Strains in which the D-lactic acid dehydrogenase gene (*ldhA* gene) in the chromosomal DNA of the *E*. *coli* BW25113 strain carrying the pKD46 plasmid is deleted were produced by the method shown below using a linear DNA comprising the chloramphenicol resistance gene *(cat* gene) and the *Bacillus subtilis-derived* levansucrase gene (*sacB* gene, GenBank accession no. BG10388).

First, the *sacB* gene was obtained by PCR as a region extending from 400 bp upstream to 200 bp downstream of the coding region. PCR was carried out using the chromosomal DNA of the *B. subtilis* 168 strain (available from various official organizations) as template, and DNAs comprising the nucleotide sequence shown in SEQ ID NO: 14 or 15 as the primer set. PCR was carried out using LA-Taq. A 25 µl reaction solution containing 100 ng of chromosomal DNA and 20 pmol each of the primer DNAs was prepared according to the enclosed LA-Taq instructions, and PCR was carried out with the following conditions: heating at 94°C for 3 minutes, followed by 30 cycles of 94°C for 15 seconds, 55°C for 20 seconds and 68°C for 1.5 minutes, and subsequent heating at 72°C for 10 minutes.

The amplified DNA fragments (*sacB* gene fragments) obtained by the above PCR were purified using a PCR purification Kit and dissolved in 50 µl of water.

Next, PCR was carried out using the pHSG399 plasmid (Takara Bio Inc.) as template, and DNAs comprising the nucleotide sequence shown in SEQ ID NO: 16 or 17 as the primer set to obtain chloramphenicol resistance gene *(cat* gene) fragments.

PCR was carried out using LA-Taq. A 25 µl reaction solution containing 40 ng of plasmid DNA and 20 pmol each of the primer DNAs was prepared according to the enclosed LA-Taq instructions, and PCR was carried out with the following conditions: heating at 94°C for 3 minutes, followed by 30 cycles of 94°C for 15 seconds, 55°C for 20 seconds and 68°C for 1.5 minutes, and subsequent heating at 72°C for 10 minutes.

The amplified DNA fragments *(cat* gene fragments) obtained by the above PCR were purified using a Gel extraction Kit and dissolved in 50 µl of water.

Next, PCR was carried out using the *sacB* gene fragments and *cat* gene fragments obtained above as templates, and DNAs comprising the nucleotide sequence shown in SEQ ID NO: 14 or 17 as the primer set. The PCR was carried out using LA-Taq. A 25 µl reaction solution containing 1 µl each of the sacB fragments and cat fragments obtained above and 20 pmol each of the primer DNAs was prepared according to the enclosed LA-Taq instructions, and PCR was carried out in the following conditions: heating at 94°C for 3 minutes, followed by 30 cycles of 94°C for 15 seconds, 55°C for 20 seconds and 68°C for 3 minutes, and subsequent heating at 72°C for 10 minutes.

The amplified DNA fragments (DNA fragments in which the *sacB* gene fragment and *cat* gene fragment are linked in the same direction) obtained by the above PCR were purified using a Gel extraction Kit and dissolved in 50 µl of water.

Next, PCR was carried out using the DNA fragments as templates, and DNAs comprising the nucleotide sequence shown in SEQ ID NO: 18 or 19 as the primer set. The DNA comprising the nucleotide sequence shown in SEQ ID NO: 18 has at its 5' end, a nucleotide sequence comprising 43 nucleotides having a sequence that is homologous to the vicinity of the 5'-end region of the *ldhA* gene, and at its 3' end, a DNA comprising the nucleotide sequence shown in SEQ ID NO: 7. The DNA comprising the nucleotide sequence shown in SEQ ID NO: 19 has, at its 5' end, a nucleotide sequence comprising 43 nucleotides that are complementary to the vicinity of the 3'-end region of the *ldhA* gene, and at its 3' end, a DNA comprising 25 nucleotides which hybridize with the nucleotide sequence shown in SEQ ID NO: 8.

PCR was carried out using EX-Taq. A 50 µl reaction solution containing 10 ng of template DNA fragments and 10 pmol each of the primer DNAs was prepared according to the enclosed EX-Taq instructions, and PCR was carried out in the following conditions: heating at 94°C for 1 minute, followed by 30 cycles of 94°C for 30 seconds, 55°C for 30 seconds and 72°C for 3 minutes, and subsequent cooling at 4°C.

It was confirmed that DNA fragments comprising, at both end, a region which is homologous to the 43 nucleotides at both ends of the *ldhA* gene and comprising the *sacB* gene and the *cat* gene (*sacB* gene + *cat* gene fragments) were amplified by PCR. The DNA fragments were subjected to agarose gel electrophoresis, then excised from the gel and purified.

Next, competent cells of the *E*. *coli* BW25113 strain carrying the pKD46 plasmid were prepared according to the method described in Example 1, and transformation was carried out using 100ng of the DNA fragments obtained above. Transformation was carried out by electroporation in a 0.1 cm cuvette (BioRad) under the conditions of 1.8 kV and 25 µF.

The transformed cells were cultured using SOC medium at 30°C for three hours. Then, the culture solution was spread on an LB agar plate containing 30 µg/ml chloramphenicol, and this was incubated at 30°C overnight. The chloramphenicol-resistant strains that grew were replicated onto a SuLB agar medium [10 g/l Bacto tryptone (Difco), 5 g/l Bacto yeast extract (Difco), 10% sucrose and 1 ml/l of 1 mol/l NaOH] plate, and sucrose-sensitive strains were selected. It was confirmed that in strains showing chloramphenicol resistance and sucrose sensitivity, the *ldhA* gene in the chromosomal DNA of the *E. coli* BW25113 strain was destroyed, and the strains were designated as the *E. coli* BW25113Δ1dhA::cat-sacB strain.

### (2) Construction of microorganism into which the L-lactic acid dehydrogenase gene has been introduced

An L-lactic acid dehydrogenase gene (lctE gene) derived from *B. subtilis* was introduced into the *ldhA* gene region in the chromosomal DNA of the *E*. *coli* BW25113Δ1dhA::cat-sacB strain produced in (1) above, and *E*. *coli* having the *lctE* gene under the regulation of the *E*. *coli ldhA* gene promoter were generated as described below.

The *lctE* gene of *B. subtilis* was obtained by PCR using the chromosomal DNA of the *B*. *subtilis* 168 strain as template and DNAs comprising the nucleotide sequence shown in SEQ ID NO: 20 or 21 as the primer set. The DNA comprising the nucleotide sequence shown in SEQ ID NO: 20 has at its 5' end, a nucleotide sequence comprising 45 nucleotides of the upstream region of the initiation codon of the *ldhA* gene, and at its 3' end, a nucleotide sequence comprising 27 nucleotides including the *lctE* gene initiation codon and its downstream region. The DNA comprising the nucleotide sequence shown in SEQ ID NO: 21 has, at its 5' end, a nucleotide sequence comprising 45 nucleotides that are complementary to the downstream region of the termination codon of the *ldhA* gene, and at its 3' end, a nucleotide sequence comprising 27 nucleotides that are complementary to a region including the *lctE* gene termination codon and its upstream region.

PCR was carried out using Pyrobest DNA polymerase (Takara Bio Inc.). A 50 µl reaction solution containing 100 ng of chromosomal DNA and 10 pmol each of the primer DNAs was prepared according to the enclosed enzyme instructions, and PCR was carried out in the following conditions: heating at 94°C for 1 minute, followed by 30 cycles of 94°C for 30 seconds, 55°C for 30 seconds and 72°C for 80 seconds, and subsequent cooling at 4°C.

It was confirmed that lctE gene fragments comprising regions that are homologous to the 45 nucleotides at the upstream or downstream region of the ldhA gene at their ends were amplified by PCR. The DNA fragments were subjected to agarose gel electrophoresis, and then excised from the gel and purified.

Competent cells of the *E. coli* BW25113Δ1dhA::cat-sacB strain carrying a pKD46 plasmid were prepared following the method described in Example 1, and transformation was carried out using 100 ng of the DNA fragments obtained above.

Transformation was carried out by electroporation in a 0.1 cm cuvette (BioRad) under the conditions of 1.8 kV and 25 µF. The transformed cells were cultured using SOC medium at 30°C for three hours. Then, the culture solution was spread on a SuLB agar medium plate, and incubated at 30°C overnight. In sucrose-resistant strains that grew, it was confirmed that the *lctE* gene is introduced into the *ldhA* gene region in the chromosomal DNA of the *E. coli* BW25113Δ1dhA::cat-sacB strain, and these strains were designated as the *E. coli* BW25113Δ1dhA::1ctE strain.

### (3) Generation of a ubiB gene-defective strain

The *E. coli* BW25113ΔubiB strain obtained in Example 1 was cultured in an LB liquid medium containing 20 mg/l kanamycin and 10 g/l glucose at 30°C overnight. Then, 0.5 ml of the obtained culture solution was mixed with 1.4 ml of fresh LB liquid medium and 100 µl of 0.1 M calcium chloride solution.

The obtained mixture was subject to culturing at 30°C for five to six hours with shaking. 400 µl of the culture was transferred to a sterilized tube, 2 µl of a P1 phage stock was added thereto, and the mixture was kept at 37°C for ten minutes. 3 ml of a soft agar solution (10 g/l Bacto tryptone, 5 g/l Bacto yeast extract, 5 mM calcium chloride, 1 ml/l of 1 mol/l NaOH and 3 g/l agar) kept at 50°C was added into the tube. The mixture was thoroughly agitated, then spread on a Ca-LB agar medium plate (10 g/l Bacto tryptone, 5 g/l Bacto yeast extract, 5 mM calcium chloride, 1 ml/l of 1 mol/l NaOH and 15 g/l agar; the plate diameter is 15 cm), and incubated at 37°C for seven hours.

Next, a portion of the soft agar was finely crushed using a spreader and collected, and then centrifuged at 1,500 g at 4°C for 10 minutes. 100 µl of chloroform was added to 1.5 ml of the obtained supernatant, and this was left at 4°C overnight and centrifuged at 15,000 g for 2 minutes. The obtained supernatant was stored at 4°C as a phage stock.

The *E. coli* BW25113Δ1dhA::lctE strain was cultured in a Ca-LB liquid medium at 30°C for 4 hours, and 200 µl of the obtained culture was centrifuged and the supernatant was removed. Then, the bacteria were suspended in 100 µl of MC buffer (0.1 mol/l MgSO₄ and 0.005 mol/l calcium chloride) to prepare a bacterial suspension.

100 µl of the phage stock obtained above was added to the bacterial suspension, and gently mixed and kept at 37°C for 10 minutes. Then, 200 µl of a 1 mol/l sodium citrate solution was added thereto, followed by centrifugation, and the bacteria were washed by removing the supernatant. The washing process was further repeated twice. Then, the product was suspended in 2 ml of an LB liquid medium and incubated at 30°C for two hours.

Next, 100 µl of the obtained culture was spread on an LB agar medium containing 30 mg/l kanamycin and 10 g/l glucose and incubated at 30°C overnight. In kanamycin-resistant strains, the *ubiB* gene was confirmed to be deleted, and these strains were designated as the *E*. *coli* BW25113ΔubiB ΔldhA::lctE strain.

### [Example 5]

### Production of L-lactic acid

The *E*. *coli* BW25113ΔubiB ΔldhA::lctE strain obtained in Example 4 was inoculated into 2 ml of an LB liquid medium containing 20 g/l glucose in a test tube and cultured at 37°C for 24 hours with shaking to obtain a culture. 50 µl of the culture was inoculated into 5 ml of a M9 liquid medium (5% glucose, 11.28 g/l M9 Minimal Salts (X5) (Difco), 100 µmol/l calcium chloride, 2 mmol/l magnesium sulfate and 10 µg/l iron sulfate) containing 6% calcium carbonate and 1% casamino acid in a test tube and cultured at 37°C for 24 hours with shaking. The culture after 24 hours was centrifuged. The supernatant was diluted 10- to 100-fold and the amounts of L-lactic acid, D-lactic acid and acetic acid that have accumulated in the culture solution were measured using F-kit D-/L-lactic acid and F-kit acetic acid from Roche Diagnostics. The results are shown in Table 3.

**Table 3**

| BACTERIAL STRAIN NAME | L-LACTIC ACID (g/l) | D-LACTIC ACID (g/l) | ACETIC ACID (g/l) |
|---|---|---|---|
| E. coli BW25113ΔubiB ΔldhA::lctE | 44. 9 | BELOW DETECTABLE LIMITS | 0.6 |

As shown in Table 3, the *E. coli* BW25113ΔubiB ΔldhA::lctE strain only produced L-lactic acid, and not D-lactic acid. It was revealed that the *ubiB* gene-defective strain also produces L-lactic acid.

### Industrial Applicability

Lactic acid can be efficiently produced according to the present invention.

### Sequence free text

SEQ ID NO: 5 - Description of Artificial Sequence: Synthetic DNA
SEQ ID NO: 6 - Description of Artificial Sequence: Synthetic DNA
SEQ ID NO: 7 - Description of Artificial Sequence: Synthetic DNA
SEQ ID NO: 8 - Description of Artificial Sequence: Synthetic DNA
SEQ ID NO: 9 - Description of Artificial Sequence: Synthetic DNA
SEQ ID NO: 10 - Description of Artificial Sequence: Synthetic DNA
SEQ ID NO: 11 - Description of Artificial Sequence: Synthetic DNA
SEQ ID NO: 12 - Description of Artificial Sequence: Synthetic DNA
SEQ ID NO: 13 - Description of Artificial Sequence: Synthetic DNA
SEQ ID NO: 14 - Description of Artificial Sequence: Synthetic DNA
SEQ ID NO: 15 - Description of Artificial Sequence: Synthetic DNA
SEQ ID NO: 16 - Description of Artificial Sequence: Synthetic DNA
SEQ ID NO: 17 - Description of Artificial Sequence: Synthetic DNA
SEQ ID NO: 18 - Description of Artificial Sequence: Synthetic DNA
SEQ ID NO: 19 - Description of Artificial Sequence: Synthetic DNA
SEQ ID NO: 20 - Description of Artificial Sequence: Synthetic DNA
SEQ ID NO: 21 - Description of Artificial Sequence: Synthetic DNA

## Claims

1. A microorganism, in which the activity of 4-hydroxybenzoate polyprenyltransferase or 2-octaprenylphenol→2-octaprenyl-6-methoxyphenol flavin reductase is reduced or lost, and which has an ability to produce lactic acid.

2. The microorganism according to claim 1, which comprises a chromosomal DNA in which a gene encoding a protein having 4-hydroxybenzoate polyprenyltransferase activity or a protein having 2-octaprenylphenol→2-octaprenyl-6-methoxyphenol flavin reductase activity is partially or completely defective.

3. The microorganism according to claim 2, wherein the gene encodes a protein comprising the amino acid sequence of SEQ ID NO: 1 or SEQ ID NO: 2; or a protein having a homology of 80% or more with the amino acid sequence of SEQ ID NO: 1 or SEQ ID NO: 2, and having 4-hydroxybenzoate polyprenyltransferase activity or 2-octaprenylphenol→2-octaprenyl-6-methoxyphenol flavin reductase activity.

4. The microorganism according to claim 2, wherein the gene is a gene comprising the nucleotide sequence of SEQ ID NO: 3 or 4; or a gene which hybridizes under stringent conditions with a polynucleotide comprising a nucleotide sequence complementary to the nucleotide sequence of SEQ ID NO: 3 or 4, and encodes a protein having 4-hydroxybenzoate polyprenyltransferase activity or 2-octaprenylphenol→2-octaprenyl-6-methoxyphenol flavin reductase activity.

5. A microorganism according to any one of claims 1 to 4, wherein the microorganism has an enhanced ability to produce lactic acid by a method selected from the following [1] to [5]:
[1] a method for partially or completely releasing at least one of the mechanisms that regulate biosynthesis of lactic acid;
[2] a method for enhancing the expression of at least one of the enzymes involved in biosynthesis of lactic acid;
[3] a method for increasing the copy number of at least one of the enzyme genes involved in biosynthesis of lactic acid;
[4] a method for partially or completely blocking at least one of the branched metabolic pathways of lactic acid biosynthesis for metabolites other than useful substances;
[5] a method for selecting a cell strain that is highly resistant to a lactic acid analogue as compared with a wild type strain.

6. The microorganism according to any one of claims 1 to 5, wherein the microorganism belongs to the genus *Erwinia, Serratia, Salmonella, Escherichia, Proteus, Pseudomonas, Acidithiobacillus, Acinetobacter, Agrobacterium, Bacillus, Bordetella, Bradyrhizobium, Brucella, Burkholderia, Caulobacter, Chloroflexus, Clostridium, Coxiella, Desulfitobacterium, Geobacter, Haemophilus, Legionella, Leptospira, Magnetococcus, Magnetospirillum, Mannheimia, Mesorhizobium, Methylobacillus, Methylobacterium, Mycobacterium, Neisseria, Nitrosomonas, Nostoc, Pasteurella, Prochlorococcus, Rhodobacter, Rhodococcus, Rhodopseudomonas, Rickettsia, Shigella, Sinorhizobium, Sphingomonas, Streptomyces, Synechococcus, Synechocystis, Vibrio, Xylella, Yersinia, Zymomonas* or *Xanthomonas.*

7. The microorganism according to any one of claims 1 to 6, wherein the microorganism is selected from the group consisting of *Erwinia berbicola, Erwinia amylovora, Erwinia carotovora, Serratia marcescens, Serratia ficaria, Serratia fonticola, Serratia liquefaciens, Salmonella enterica, Salmonella enteritidis, Salmonella paratyphi, Salmonella typhi, Salmonella dublin, Salmonella typhimurium, Escherichia coli, Proteus rettgeri, Pseudomonas putida, Pseudomonas fluorescens, Pseudomonas aeruginosa, Pseudomonas dacunhae, Pseudomonas fluorescens, Pseudomonas syringae, Pseudomonas thazdinophilum, Acidithiobacillus ferrooxidans, Acinetobacter sp.* ATCC 33305, *Agrobacterium tumefaciens, Bacillus halodurans, Bordetella bronchiseptica, Bordetella parapertussis, Bordetella pertussis, Bradyrhizobium japonicum, Brucella melitensis, Burkholderia pseudomallei, Caulobacter crescentus, Chloroflexus aurantiacus, Clostridium acetobutylicum, Clostridium botulinum, Clostridium difficile, Coxiella burnetii, Desulfitobacter iumhafniense, Geobacter metallireducens, Haemophilus ducreyi, Legionella pneumophila, Leptospira interrogans, Magnetococcus* MC-1, *Magnetospirillum magnetotacticum, Mannheimia haemolytica, Mesorhizobium loti, Methylobacillus flagellatus, Methylobacterium extorquens, Mycobacterium bovis, Mycobacterium leprae, Mycobacterium marinum, Mycobacterium tuberculosis, Neisseria gonorrhoeae, Neisseria meningitidis, Nitrosomonas europaea, Nostoc punctiforme, Pasteurella multocida, Prochlorococcus marinus, Rhodobacter capsulatus, Rhodococcus sp.* strain 124, *Rhodopseudomonas palustris, Rickettsia prowazekii, Shigella flexneri, Sinorhizobium meliloti, Sphingomonas aromaticivorans, Streptomyces avermitilis, Streptomyces coelicolor, Synechococcus sp.* WH8102, *Synechocystis sp.* PCC6803, Vi*brio cholerae, Xylella fastidiosa, Xylella fastidiosa, Yersinia pestis, Zymomonas mobilis, Xanthomonas ovyzae* and *Xanthomonas capestris.*

8. A process for producing lactic acid, which comprises culturing the microorganism according to any one of claims 1 to 7 in a medium so as to produce and accumulate lactic acid in the culture, and recovering lactic acid from the culture.
